# EUROPEAN PATENT SPECIFICATION

(11) **EP 4 021 629 B1**
(45) Date of publication and mention of the grant of the patent: **04.12.2024**
(21) Application number: 20760839.9
(22) Date of filing: 21.08.2020
(51) Int. Cl.: B01J 13/16, A01N 25/28, A61K 8/11, A61K 9/48, A61Q 19/00, A61Q 19/10, C09B 67/02, C11D 3/50, C11D 17/00

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
PERFECTIONNEMENTS APPORTÉS AUX COMPOSÉS ORGANIQUES OU EN RELATION AVEC CEUX-CI

(30) Priority: 29.08.2019 GB 201912382
(43) Date of publication of application: 06.07.2022
(73) Proprietor: Givaudan SA, 1214 Vernier (CH)
(72) Inventor: AUSSANT, Emmanuel, 75011 Paris (FR); BENAROUS, Nawal, 95190 Goussainville (FR); GUINEBRETIERE, Sandra, 95130 Franconville (FR); POELS, Eduard, 18697 Almuñécar (ES)
(74) Representative: Global Patents
(86) International application number: PCT/EP2020/073462
(87) International publication number: WO 2021/037703

(56) References cited:
- WO-A1-2016/193435
- WO-A1-2018/006089
- WO-A1-2019/063515
- US-A1- 2013 330 292
- US-A1- 2018 015 009

## Description

The present invention is concerned with encapsulated compositions comprising at least one core-shell microcapsule. The invention also relates to a method for preparing such compositions and to their use to enhance the performance of a benefit agent in a consumer product.

It is known to incorporate encapsulated benefit agents in consumer products, such as household care, personal care and fabric care products. Benefit agents include for example fragrances, cosmetic agents, food ingredients, nutraceuticals, drugs and substrate enhancers.

Microcapsules that are particularly suitable for delivery of such benefit agents are core-shell microcapsules, wherein the core usually comprises the benefit agent and the shell is impervious or partially impervious to the benefit agent. Generally, these microcapsules are employed in aqueous media and the encapsulated benefit agents are hydrophobic. A broad selection of shell materials can be used, provided the shell material is impervious or partially impervious to the encapsulated benefit agent.

Benefit agents are encapsulated for a variety of reasons. Microcapsules can isolate and protect such materials from external suspending media, such as consumer product bases, in which they may be incompatible or unstable. They are also used to assist in the deposition of benefit agents onto substrates, such as skin or hair, or also fabrics or hard household surfaces in case of perfume ingredients. They can also act as a means of controlling the spatio-temporal release of a benefit agent.

A wide variety of encapsulating media as well as benefit agents suitable for the preparation of encapsulated compositions has been proposed in the prior art. Such encapsulating media include synthetic resins made from polyamides, polyureas, polyurethanes, polyacrylates, melamine-derived resins, or mixtures thereof.

In many instances, deposition and adherence of such microcapsules on smooth surfaces and especially on keratinous surfaces, such as skin and hair, are insufficient and the expected benefits associated with the use of microcapsules not optimal. This is especially the case for rinse-off products involving large amounts of water. In this case, a lack of deposition may be due to dilution of the microcapsules. Large volumes of rinse water may also wash off the microcapsules from the surface.

WO 2011/161229 A1 describes encapsulated fragrance compositions comprising core-shell microcapsules with a shell made of a polyurea resin formed by reaction of two structurally different isocyanates. These microcapsules show good deposition on keratinous surfaces in rinse-off applications. Documents WO 2016/193435 A1, US 2018/015009 A1 and WO 2018/006089 A1 disclose core-shell microcapsules.

However, it has been found that especially in hair care the method of drying after rinsing can have a significant effect on benefits brought by the microcapsules. While good deposition and adherence were generally observed with air drying, using a hair dryer turned out to result in reduced performance.

It is therefore a problem underlying the present invention to overcome the above-mentioned shortcomings in the prior art. In particular, it is a problem underlying the present invention to provide encapsulated compositions of the above-mentioned kind that show improved deposition and adherence on hair after drying the same with a hair dryer. These microcapsules should keep their desired benefit-agent release properties, during manufacture, storage and in application. Furthermore, the compositions should be producible in an operationally safe, robust and cost-efficient process.

These problems are solved by an encapsulated composition according to the present invention. Such a composition comprises at least one core-shell microcapsule. The at least one core-shell microcapsule comprises a core comprising at least one benefit agent and a shell surrounding the core. The shell comprises a polyurea resin formed by reaction of at least one trifunctional isocyanate with at least one polyfunctional amine not being chitosan. The at least one trifunctional isocyanate is an adduct of an aliphatic triol with at least one araliphatic diisocyanate. The weight ratio between moieties of the polyurea resin, which are derived from the trifunctional isocyanate, and the core is between 0.09 and 0.30, preferably between 0.10 and 0.20, more preferably between 0.11 and 0.18. The shell additionally comprises chitosan.

In the present context, the term *"benefit agent"* refers to any substance which, when added to a product, may improve the perception of this product by a consumer or may enhance the action of this product in an application. Typical benefit agents include perfume ingredients, flavor ingredients, cosmetic ingredients, bioactive agents (such as bactericides, insect repellents and pheromones), substrate enhancers (such as silicones and brighteners), enzymes (such as lipases and proteases), dyes, pigments and nutraceuticals.

Organic isocyanates are compounds in which an isocyanate group is bonded to an organic residue (R-N=C=O or R-NCO). In the context of the present invention, polyisocyanates (or polyfunctional isocyanates) are organic isocyanates with two or more (e.g. 3, 4, 5, etc.) isocyanate groups in a molecule. A trifunctional isocyanate is an isocyanate with three isocyanate groups in a molecule.

As known in the art, chitosan is a biopolymer derived from chitin, forming the exoskeleton of crustaceans and preserving the shape of various fungi, such as *Ascomycetes*, *Zygomycetes*, *Basidiomycetes* and *Deuteromycetes*, for example *Absidia*, *Mucor*, *Aspergillus Niger*, *Ganoderma Lucidum* and *Rhizopus Oryzae.* Chitosan production involves the alkaline or enzymatic deacetylation of chitin and is characterized by a deacetylation grade. Both low deacetylation grades, typically below 80 % deacetylation, and high deacetylation grades, typically higher than or equal to 80% deacetylation exist. Deacetylated chitosan is a copolymer consisting of N-acetyl-D-glucosamine and D-glucosamine moieties. The deacetylation grade may be determined by ¹H and/or ¹³C nuclear magnetic resonance spectroscopy. Chitosan is available with molecular weights typically ranging from 3'000 and 5'000'000 g/mol. The molecular weight may be determined by viscosity measurement and/or gel permeation chromatography, according to methods known in the art.

In context of the present invention, the term *"polyfunctional amine not being chitosan"* denotes amines that comprise at least two groups capable of reacting with NCO groups, wherein at least one of the groups capable of reacting with NCO groups is a primary or secondary amino group. These amines are structurally not identical with chitosan. When the polyfunctional amine contains only one primary or secondary amino group, it will contain one or more additional functional groups that are capable of reacting with NCO groups in a polymerization reaction. The groups of the polyfunctional amines that are reactive toward NCO groups are preferably chosen from hydroxyl groups and primary or secondary amino groups. Reaction of NCO groups with amino groups leads to the formation of urea groups. Reaction of NCO groups with OH groups leads to the formation of urethane groups. However, the reaction with OH groups often requires a catalyst. The amount of polyfunctional amines, which is introduced, is usually in a molar excess relative to the stoichiometric amount needed to convert the free isocyanate groups.

Generally, *"araliphatic isocyanates or polyisocyanates"* are compounds in which at least one isocyanate group is bonded to an aromatic residue through an alkyl residue, in particular a methylene residue.

In order to avoid any ambiguity, that the *"shell additionally comprises chitosan"* does not necessarily mean that, during encapsulation, the chitosan undergoes a reaction with other shell-forming materials, in particular with the at least one trifunctional isocyanate, to build up the polyurea resin, although exactly this might well be the case, as explained further herein below. More specifically, the present invention also encompasses cases where microcapsule shells for instance comprise a coating of chitosan, meaning that that the chitosan is deposited on the shells. On the other hand, the chitosan can also be entrapped in the shells of the microcapsules.

In addressing the problems of the prior art, it has been found that using a specific amount of at least one trifunctional isocyanate in combination with chitosan in the formation polyurea microcapsule shells results in improved deposition and adherence of the capsules on hair after drying the same with a hair dryer.

The aliphatic triol can be selected from the group consisting of 2-ethylpropane-1,2,3-triol and 2-ethyl-2-(hydroxymethyl)propane-1,3-diol, preferably 2-ethylpropane-1,2,3-triol.

Preferably, the at least one araliphatic diisocyanate is selected from the group consisting of 1,2-bis(isocyanatomethyl)benzene, 1,3-bis(isocyanatomethyl) benzene, 1,4-bis(isocyanatomethyl)benzene, 1-isocyanato-2-(isocyanatomethyl) benzene, 1-isocyanato-3-(isocyanatomethyl)benzene and 1-isocyanato-4-(isocyanatomethyl)benzene.

Trifunctional isocyanates of that kind are available under the Trade Marks Takenate D-110N (ex Mitsui) or Desmodur Quix 175 (ex Covestro). Such isocyanates have the advantage to be more reactive than aliphatic polyisocyanates and less reactive than aromatic polyisocyanates. Without being bound by any theory, it can be assumed that, due this intermediate reactivity, the polyurea resin formed during the polyaddition of araliphatic polyisocyanate with polyfunctional amines are more homogeneously crosslinked than those obtained with aromatic polyisocyanate. On the other hand, the cross-linking is more efficient than that obtained with aliphatic polyisocyanates.

In a particular embodiment of the present invention, the trifunctional isocyanate is an adduct of 2-ethylpropane-1,2,3-triol and 1,3-bis(isocyanatomethyl) benzene.

As mentioned before, in a preferred embodiment of the present invention, the chitosan is deposited on an outer surface of the shell surrounding the core and/or the chitosan is entrapped in the shell surrounding the core. In this context, the composition can additionally comprise free chitosan, as adsorption/desorption-equilibria of chitosan may occur.

In the context of the present invention, the term *"free chitosan"* is used to describe chitosan which, when the microcapsules are dispersed in a dispersing medium, is separated spatially from the microcapsules by the dispersing medium.

On the other hand, the polyurea resin can also be formed by reaction of the at least one trifunctional isocyanate with the at least one polyfunctional amine not being chitosan and with the chitosan. Without being bound by any theory, the applicant believes that using chitosan as a reactive species in the formation of the polyurea resin makes this resin more viscoelastic, which, in turn, promotes the adherence of the microcapsule shell with substrates.

In another preferred embodiment of the present invention, the polyurea resin is formed by reaction of the at least one trifunctional isocyanate and a water-dispersible polyisocyanate with at least one polyfunctional amine not being chitosan, and optionally with the chitosan.

In the present context, *"water-dispersible polyisocyanate"* refers to polyisocyanates that can be dispersed in the form of finely divided solid particles or liquid droplets in aqueous media.

Suitable water-dispersible polyisocyanates are, for instance, aromatic, alicyclic or aliphatic.

The water-dispersible polyisocyanate is preferably an anionically modified polyisocyanate. Anionically modified polyisocyanates comprise at least two isocyanate groups and at least one functional group which is anionic or anionogenic. An *"anionogenic functional group"* is a group which can become anionic depending on the chemical environment, for instance the pH. Suitable anionic or anionogenic groups are, for instance, carboxylic acid groups, sulfonic acid groups, phosphonic acid groups and salts thereof.

The anionically modified polyisocyanate can comprise one or more sulfonic acid groups or salts thereof. Suitable salts can be sodium, potassium or ammonium salts. Ammonium salts are preferred.

Preferably, the anionically modified polyisocyanate is obtained by reaction of a polyisocyanate with 2-(cyclohexylamino)-ethanesulfonic acid and/or 3-(cyclohexylamino)-propanesulfonic acid.

More preferably, the anionically modified polyisocyanate is obtained by reaction of a polyisocyanate with 2-(cyclohexylamino)-ethanesulfonic acid and/or 3-(cyclohexylamino)-propanesulfonic acid, wherein the polyisocyanate is selected from hexamethylene diisocyanate, tetramethylene diisocyanate, isophorone diisocyanate, dicyclohexylmethane-4,4'-diisocyanate, 2,4- and 2,6-toluylene diisocyanate and isomer mixtures thereof, diphenylmethane diisocyanates, biurets, allophanates and/or isocyanurates of the before-mentioned polyisocyanates.

The anionically modified polyisocyanate can be selected in each case from anionically modified hexamethylene diisocyanate, isophorone diisocyanate, dicyclohexylmethane-4,4'-diisocyanate, the isocyanurate of hexamethylene diisocyanate and mixtures thereof.

Preferably, the anionically modified polyisocyanate has:
- an average isocyanate functionality of at least 1.8,
- a content of isocyanate groups (calculated as NCO; molecular weight = 42) of 4.0 to 26.0 wt.-%,
- a content of sulfonate groups (calculated as SO₃; molecular weight = 80) of 0.1 to 7.7 wt.-% and
- optionally a content of ethylene oxide units bonded within polyether chains (calculated as C₂H₂O; molecular weight = 44) of 0 to 19.5 wt.-%, wherein the polyether chains contain a statistical average of 5 to 55 ethylene oxide units.

In a preferred embodiment of the present invention, the water-dispersible polyisocyanate is a water-dispersible polyisocyanate based on hexamethylene diisocyanate. More specifically, the anionically modified polyisocyanate can be selected from an anionically modified hexamethylene diisocyanate, an anionically modified isocyanurate of hexamethylene diisocyanate and mixtures thereof.

In a particularly preferred embodiment, the anionically modified polyisocyanate is according to Formula (1).

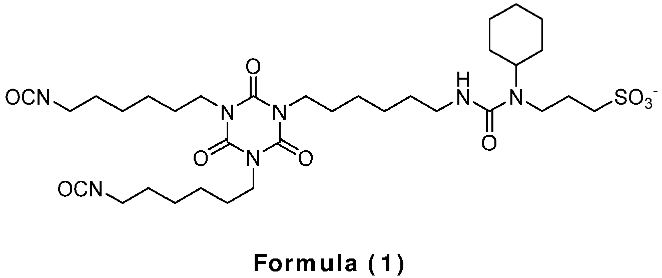

Formula (1) shows a commercially available anionically modified polyisocyanate, which is a modified isocyanurate of hexamethylene diisocyanate, sold by Covestro under the trademark Bayhydur XP2547.

The weight ratio between moieties of the polyurea resin, which are derived from the water-dispersible polyisocyanate, and the core can be between 0.001 and 0.2, preferably between 0.005 and 0.1, more preferably between 0.01 and 0.05.

The polyfunctional amine not being chitosan is preferably selected from diamines, triamines, tetramines, and higher order polyfunctional amines, aminoalcohols, melamines, urea, hydrazines, polymeric polyamines, and mixtures thereof.

Suitable diamines are, for example, 1,2-ethylenediamine, 1,3-propylenediamine, 1,4-diaminobutane, 1,5-diaminopentane, 1,6-diaminohexane, 1,3-diamino-1-methylpropane, 1,4-diaminocyclohexane, piperazin or mixtures thereof.

Suitable amino alcohols are, for example, 2-aminoethanol, 2-(N-methylamino)ethanol, 3-aminopropanol, 4-aminobutanol, 1-ethylaminobutan-2-ol, 2-amino-2-methyl-1-propanol, 4 methyl-4-aminopentan-2-ol or mixtures thereof.

Suitable polymeric polyamines are in principle linear or branched polymers that have at least two primary or secondary amino groups. Additionally, these polymers can have tertiary amino groups in the polymer chain.

The polymeric polyamines are preferably selected from polyalkyleneamines, polyvinylamines, polyetheramines and mixtures thereof. More preferably, the polymeric polyamines are selected from poly(alkyleneimines), in particular poly(ethyleneimines).

Preference is given to polymeric polyamines having a weight-average molecular weight of at least 300 g/mol. More preferred are polymeric polyamines having a weight-average molecular weight of from 500 to 2'000'000 g/mol, in particular from 700 to 1'000'000 g/mol, even more particularly from 800 to 500'000 g/mol.

In a preferred embodiment, the polyfunctional amine not being chitosan is a poly(ethyleneimine).

Poly(ethyleneimines) may be short chain poly(ethyleneimines) with the general formula H₂N(CH₂CH₂NH)ₙH, wherein n is an integer > 1 (n = 2: diethylenetriamine; n = 3: triethylenetetramine; n = 4: tetraethylenepentamine). These are sometimes called poly(ethyleneamines) or poly(alkylenepolyamines). Poly(ethyleneimines) may also be long chain poly(ethyleneimines).

According to the present invention, poly(ethyleneimines) with a molecular weight of at least 500 g/mol, preferably from 600 to 30'000 or 650 to 25'000 g/mol and in particular from 700 to 10'000 g/mol or 850 to 5'000 g/mol, are preferably used.

The polyfunctional amine can be a poly(ethyleneimine) containing the following repeat units 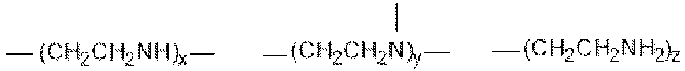 wherein
- x is from 8 to 1'500, preferably from 10 to 1'000;
- y is from 0 to 10, preferably from 0 to 5, especially 0;
- z is 2+ y.

With these poly(ethyleneimines) good results could be achieved, in particular with respect to leakage in extractive media.

Preferred poly(ethyleneimines) are linear poly(ethyleneimines), wherein x is from 8 to 1500, y is 0 and z is 2.

Preferred commercially available poly(ethylenimines) are sold by BASF SE under the trademark Lupasol, particularly Lupasol G100.

The weight ratio between moieties of the polyurea resin, which are derived from the polyfunctional amine, and the core can be between 0.01 and 0.1, preferably between 0.015 and 0.05, more preferably between 0.018 and 0.025.

In a preferred embodiment of the present invention, the shell additionally comprises a cationic polymer. On one hand, cationic polymers are known to improve the deposition and rinse resistance of microcapsules on various substrates, such as fabrics, skin and hair. On the other hand, the cationic polymer can also act as a dispersion aid.

The cationic polymer can be an ampholytic polymer. In the context of the present invention, an *"ampholytic polymer"* is to be understood as a polymer comprising both cationic and anionic groups, or comprising corresponding ionizable groups. The cationic ampholytic polymer comprises more cationic groups than anionic groups or groups that can form anions, and as such, has a net positive charge.

The ampholytic polymer can comprise from 1 to 99 mol-% of cationic groups and from 1 to 99 mol-% of anionic groups or groups than can form an anion. In a preferred embodiment of the present invention, the ampholytic polymer comprises 2 to 99 mol-%, in particular 30 to 95 mol-%, and more particularly 60 to 90 mol-%, of cationic groups and 1 to 98 mol-%, in particular 5 to 70 mol-%, and more particularly 10 to 40 mol-% of anionic groups or groups than can form an anion.

The cationic groups in the cationic polymer can be pH independent. The cationic groups in the cationic polymer can be quaternary ammonium groups.

The cationic polymer can be derived from at least one a monomer bearing quaternary ammonium functionality. In particular, the cationic monomer can be selected from the group consisting of quaternized dimethylaminoethyl acrylate (ADAME), quaternized dimethylaminoethyl methacrylate (MADAME), dimethyldiallyl ammonium chloride (DADMAC), acrylamidopropyltrimethylammonium chloride (APTAC) and methacrylamidopropyltrimethylammonium chloride (MAPTAC).

When the cationic polymer comprises anionic groups or groups that can form anions, it can be additionally derived from a monomer selected from the group consisting of acrylic based monomers, including acrylic acid, methacrylic acid, itaconic acid, crotonic acid, maleic acid, fumaric acid and strong-acid monomers, for example monomers with a sulfonic or a phosphonic acid-type function such as 2-acrylamido-2-methylpropane sulfonic acid, vinylsulfonic acid, vinylphosphonic acid, allylsulfonic acid, allylphosphonic acid, styrene sulfonic acid. The acrylic based monomer may also be any water-soluble salts of these monomers, wherein the salt is a salt of an alkali metal, an alkaline-earth metal or an ammonium. The most preferred acrylic based monomer is acrylic acid, methacrylic acid, or a water soluble salt thereof.

The cationic polymer can further be additionally derived from a non-ionic monomer selected from the group consisting of water soluble vinyl monomers, more particularly acrylamide, methacrylamide, N-isopropylacrylamide, N,N-dimethylacrylamide, N-methylolacrylamide, N-vinylformamide, N-vinyl acetamide, N-vinylpyridine and/or N-vinylpyrrolidone.

The cationic polymer can be an ampholytic co-polymer derived from a cationic monomer or a monomer that can form cations, in particular containing at least one quaternary ammonium group, an anionic monomer or a monomer that can form anions, in particular based on acrylic acid, methacrylic acid or a derivative thereof, and optionally a non-ionic monomer. Such polymers offer an optimal combination of being compatible with the shell, having good dispersion efficiency, good flow properties and excellent affinity with the various substrates hereinabove mentioned.

In a more particular embodiment, the ampholytic co-polymer is a co-polymer of acrylic acid or methacrylic acid, and acrylamidopropyltrimethylammonium chloride (APTAC) or methacrylamidopropyltrimethylammonium chloride (MAPTAC), as for instance described in WO 2016/207180 A1 or WO 2016/207187 A1.

In a still more particular embodiment, the ampholytic copolymer is a terpolymer formed from acrylic acid monomer, MAPTAC monomer and acrylamide monomer.

In a more preferred embodiment, the acrylic acid/MAPTAC copolymer, and more particularly the terpolymer, is formed by reacting 1 to 2 molar equivalents of acrylic acid monomer with 4 molar equivalents of the MAPTAC monomer, more particularly 1 molar equivalent of acrylic acid monomer to 4 molar equivalents of MAPTAC monomer (for example Floset CAPS 371L), and still more particularly 1.6 molar equivalents of acrylic acid monomer to 4 molar equivalents of MAPTAC monomer.

Although, in the context of the present invention, Floset CAPS 371L is a preferred cationic polymer, it can also be replaced by any other acrylic acid/MAPTAC copolymer described in the previous paragraph. Another preferred cationic polymers based on MAPTAC/acrylamide is Salcare SC60, commercialized by BASF. In an embodiment of the invention the copolymer has a molecular weight of at least 100'000 g/mol, and more particularly at least 500'000 g/mol.

Polymers with lower molecular weight do not provide the desired performance, while polymers having higher molecular weight increase the viscosity of both emulsion and microcapsule dispersions to a too large extent.

The weight ratio of the ampholytic polymer to the core can be between 0.04 and 0.20, preferably between 0.07 and 0.15, more preferably between 0.8 and 0.12.

The ampholytic polymer can be prepared using polymerization techniques that are well known to a person skilled in the art. These known polymerization techniques include solution polymerization, gel polymerization, precipitation polymerization, inverse emulsion polymerization, aqueous emulsion polymerization, suspension polymerization and micellar polymerization.

Preferably, the weight ratio between the chitosan and/or moieties of the polyurea resin, which are derived from the chitosan, and the core is between 0.002 and 0.01, preferably between 0.004 and 0.008, more preferably between 0.006 and 0.007. The level of chitosan is limited by its solubility under the conditions prevailing during microencapsulation. If the level of chitosan is too high, then part of this polymer will precipitate in the slurry of microcapsules. Conversely, if the level of chitosan is too low, then the desired benefit is inexistent.

The chitosan can have an average molecular weight from of 5'000 to 1'000'000 g/mol, preferably from 7'500 to 500'000 g/mol, still more preferably from 10'000 to 250'000 g/mol. If the molecular weight of the chitosan is too high, the microcapsule may agglomerate. If the molecular weight of the chitosan is too low, deposition and adhesion of the microcapsules on both keratinous surfaces may be insufficient.

The chitosan can have an average deacetylation grade of higher than 60 %, preferably higher than 70 %, more preferably higher than 80 %. Chitosan with such high deacetylation grades are more soluble than chitosan having lower acetylation grades and therefore more suitable for the sake of the present invention.

In context of the present invention, the source of chitosan can be selected form crustaceans (i.e. shrimp chitosan) or fungi (i.e. mushroom chitosan). The use of mushroom chitosan has the advantage that microcapsules can be obtained that are entirely of non-animal origin, or in other words vegan. Some consumers tend to favor materials that are of entirely of non-animal origin.

The benefit agent can be selected from the group consisting of at least one perfume ingredient and at least one cosmetic ingredient.

The at least one perfume ingredient can be selected from the group consisting of ADOXAL^{™} (2,6,10-trimethylundec-9-enal); AGRUMEX^{™} (2-(tert-butyl)cyclohexyl acetate); ALDEHYDE C 10 DECYLIC (decanal); ALDEHYDE C 11 MOA (2-methyldecanal); ALDEHYDE C 11 UNDECYLENIC (undec-10-enal); ALDEHYDE C 110 UNDECYLIC (undecanal); ALDEHYDE C 12 LAURIC (dodecanal); ALDEHYDE C 12 MNA PURE (2-methylundecanal); ALDEHYDE ISO C 11 ((E)-undec-9-enal); ALDEHYDE MANDARINE 10%/TEC ((E)-dodec-2-enal); ALLYL AMYL GLYCOLATE (allyl 2-(isopentyloxy)acetate); ALLYL CYCLOHEXYL PROPIONATE (allyl 3-cyclohexylpropanoate); ALLYL OENANTHATE (allyl heptanoate); AMBER CORE^{™} (1-((2-(tert-butyl)cyclohexyl)oxy)butan-2-ol); AMBERMAX^{™} (1,3,4,5,6,7-hexahydro-beta,1,1,5,5-pentamethyl-2H-2,4a-methanonaphthal-ene-8-ethanol); AMYL SALICYLATE (pentyl 2-hydroxybenzoate); APHERMATE (1-(3,3-dimethylcyclohexyl)ethyl formate); BELAMBRE^{™} ((1R,2S,4R)-2'-isopropyl-1,7,7-trimethylspiro[bicyclo[2.2.1]heptane-2,4'-[1,3]dioxane]); BIGARYL (8-(sec-butyl)-5,6,7,8-tetrahydroquinoline); BOISAMBRENE FORTE^{™} ((ethoxymethoxy)cyclododecane); BOISIRIS^{™} ((1S,2R,5R)-2-ethoxy-2,6,6-trimethyl-9-methylenebicyclo[3.3.1]nonane); BORNYL ACETATE ((2S,4S)-1,7,7-trimethylbicyclo[2.2.1]heptan-2-yl acetate); BUTYL BUTYRO LACTATE (1-butoxy-1-oxopropan-2-yl butyrate); BUTYL CYCLOHEXYL ACETATE PARA (4-(tert-butyl)cyclohexyl acetate); CARYOPHYLLENE ((Z)-4,11,11-trimethyl-8-methylenebicyclo[7.2.0]undec-4-ene); CASHMERAN^{™} (1,1,2,3,3-pentamethyl-2,3,6,7-tetrahydro-1H-inden-4(5H)-one); CASSYRANE^{™} (5-tert-butyl-2-methyl-5-propyl-2H-furan); CITRAL ((E)-3,7-dimethylocta-2,6-dienal); CITRAL LEMAROME^{™} N ((E)-3,7-dimethylocta-2,6-dienal); CITRATHAL^{™} R ((Z)-1,1-diethoxy-3,7-dimethylocta-2,6-diene); CITRONELLAL (3,7-dimethyloct-6-enal); CITRONELLOL (3,7-dimethyloct-6-en-1-ol); CITRONELLYL ACETATE (3,7-dimethyloct-6-en-1-yl acetate); CITRONELLYL FORMATE (3,7-dimethyloct-6-en-1-yl formate); CITRONELLYL NITRILE (3,7-dimethyloct-6-enenitrile); CITRONELLYL PROPIONATE (3,7-dimethyloct-6-en-1-yl propionate); CLONAL (dodecanenitrile); CORANOL (4-cyclohexyl-2-methylbutan-2-ol); COSMONE^{™} ((Z)-3-methylcyclotetradec-5-enone); CYCLAMEN ALDEHYDE (3-(4-isopropylphenyl)-2-methylpropanal); CYCLOGALBANATE (allyl 2-(cyclohexyloxy) acetate); CYCLOHEXYL SALICYLATE (cyclohexyl 2-hydroxybenzoate); CYCLOMYRAL (8,8-dimethyl-1,2,3,4,5,6,7,8-octahydronaphthalene-2-carbaldehyde); DAMASCENONE ((E)-1-(2,6,6-trimethylcyclohexa-1,3-dien-1-yl)but-2-en-1-one); DAMASCONE ALPHA ((E)-1-(2,6,6-trimethylcyclohex-2-en-1-yl)but-2-en-1-one); DAMASCONE DELTA ((E)-1-(2,6,6-trimethylcyclohex-3-en-1-yl)but-2-en-1-one); DECENAL-4-TRANS ((E)-dec-4-enal); DELPHONE (2-pentylcyclopentanone); DIHYDRO ANETHOLE (propanedioic acid 1-(1-(3,3-dimethylcyclohexyl)ethyl) 3-ethyl ester); DIHYDRO JASMONE (3-methyl-2-pentylcyclopent-2-enone); DIMETHYL BENZYL CARBINOL (2-methyl-1-phenylpropan-2-ol); DIMETHYL BENZYL CARBINYL ACETATE (2-methyl-1-phenylpropan-2-yl acetate); DIMETHYL BENZYL CARBINYL BUTYRATE (2-methyl-1-phenylpropan-2-yl butyrate); DIMETHYL OCTENONE (4,7-dimethyloct-6-en-3-one); DIMETOL (2,6-dimethylheptan-2-ol); DIPENTENE (1-methyl-4-(prop-1-en-2-yl)cyclohex-1-ene); DUPICAL^{™} ((E)-4-((3aS,7aS)-hexahydro-1H-4,7-methanoinden-5(6H)-ylidene)butanal); EBANOL^{™} ((E)-3-methyl-5-(2,2,3-trimethylcyclopent-3-en-1-yl)pent-4-en-2-ol); ETHYL CAPROATE (ethyl hexanoate); ETHYL CAPRYLATE (ethyl octanoate); ETHYL LINALOOL ((E)-3,7-dimethylnona-1,6-dien-3-ol); ETHYL LINALYL ACETATE ((Z)-3,7-dimethylnona-1,6-dien-3-yl acetate); ETHYL OENANTHATE (ethyl heptanoate); ETHYL SAFRANATE (ethyl 2,6,6-trimethylcyclohexa-1,3-diene-1-carboxylate); EUCALYPTOL ((1s,4s)-1,3,3-trimethyl-2-oxabicyclo[2.2.2]octane); FENCHYL ACETATE ((2S)-1,3,3-trimethylbicyclo[2.2.1]heptan-2-yl acetate); FENCHYL ALCOHOL ((1S,2R,4R)-1,3,3-trimethylbicyclo[2.2.1]heptan-2-ol); FIXOLIDE^{™} (1-(3,5,5,6,8,8-hexamethyl-5,6,7,8-tetrahydronaphthalen-2-yl)ethanone); FLORALOZONE^{™} (3-(4-ethylphenyl)-2,2-dimethylpropanal); FLORHYDRAL (3-(3-isopropylphenyl)butanal); FLOROCYCLENE^{™} ((3aR,6S,7aS)-3a,4,5,6,7,7a-hexahydro-1H-4,7-methanoinden-6-yl propionate); FLOROPAL^{™} (2,4,6-trimethyl-4-phenyl-1,3-dioxane); FRESKOMENTHE^{™} (2-(sec-butyl)cyclohexanone); FRUITATE ((3aS,4S,7R,7aS)-ethyl octahydro-1H-4,7-methanoindene-3a-carboxylate); FRUTONILE (2-methyldecanenitrile); GALBANONE^{™} PURE (1-(3,3-dimethylcyclohex-1-en-1-yl)pent-4-en-1-one); GARDOCYCLENE^{™} ((3aR,6S,7aS)-3a,4,5,6,7,7a-hexahydro-1H-4,7-methanoinden-6-yl isobutyrate); GERANIOL ((E)-3,7-dimethylocta-2,6-dien-1-ol); GERANYL ACETATE SYNTHETIC ((E)-3,7-dimethylocta-2,6-dien-1-yl acetate); GERANYL ISOBUTYRATE ((E)-3,7-dimethylocta-2,6-dien-1-yl isobutyrate); GIVESCONE^{™} (ethyl 2-ethyl-6,6-dimethylcyclohex-2-enecarboxylate); HABANOLIDE^{™} ((E)-oxacyclohexadec-12-en-2-one); HEDIONE^{™} (methyl 3-oxo-2-pentylcyclopentaneacetate); HERBANATE^{™} ((2S)-ethyl 3-isopropylbicyclo[2.2.1]hept-5-ene-2-carboxylate); HEXENYL-3-CIS BUTYRATE ((Z)-hex-3-en-1-yl butyrate); HEXYL CINNAMIC ALDEHYDE ((E)-2-benzylideneoctanal); HEXYL ISOBUTYRATE (hexyl isobutyrate); HEXYL SALICYLATE (hexyl 2-hydroxybenzoate); INDOFLOR^{™} (4,4a,5,9b-tetrahydroindeno[1,2-d][1,3]dioxine); IONONE BETA ((E)-4-(2,6,6-trimethylcyclohex-1-en-1-yl)but-3-en-2-one); IRISONE ALPHA ((E)-4-(2,6,6-trimethylcyclohex-2-en-1-yl)but-3-en-2-one); IRONE ALPHA ((E)-4-(2,5,6,6-tetramethylcyclohex-2-en-1-yl)but-3-en-2-one); ISO E SUPER^{™} (1-(2,3,8,8-tetramethyl-1,2,3,4,5,6,7,8-octahydronaphthalen-2-yl)ethanone); ISOCYCLOCITRAL (2,4,6-trimethylcyclohex-3-enecarbaldehyde); ISONONYL ACETATE (3,5,5-trimethylhexyl acetate); ISOPROPYL METHYL-2-BUTYRATE (isopropyl 2-methyl butanoate); ISORALDEINE^{™} 70 ((E)-3-methyl-4-(2,6,6-trimethylcyclohex-2-en-1-yl)but-3-en-2-one); JASMACYCLENE^{™} ((3aR,6S,7aS)-3a,4,5,6,7,7a-hexahydro-1H-4,7-methanoinden-6-yl acetate); JASMONE CIS ((Z)-3-methyl-2-(pent-2-en-1-yl)cyclopent-2-enone); KARANAL^{™} (5-(sec-butyl)-2-(2,4-dimethylcyclohex-3-en-1-yl)-5-methyl-1,3-dioxane); KOAVONE ((Z)-3,4,5,6,6-pentamethylhept-3-en-2-one); LEAF ACETAL ((Z)-1-(1-ethoxyethoxy)hex-3-ene); LEMONILE^{™} ((2E,6Z)-3,7-dimethylnona-2,6-dienenitrile); LIFFAROME^{™} GIV ((Z)-hex-3-en-1-yl methyl carbonate); LILIAL^{™} (3-(4-(tert-butyl)phenyl)-2-methylpropanal); LINALOOL (3,7-dimethylocta-1,6-dien-3-ol); LINALYL ACETATE (3,7-dimethylocta-1,6-dien-3-yl acetate); MAHONIAL^{™} ((4E)-9-hydroxy-5,9-dimethyl-4-decenal); MALTYL ISOBUTYRATE (2-methyl-4-oxo-4H-pyran-3-yl isobutyrate); MANZANATE (ethyl 2-methylpentanoate); MELONAL^{™} (2,6-dimethylhept-5-enal); MENTHOL (2-isopropyl-5-methylcyclohexanol); MENTHONE (2-isopropyl-5-methylcyclohexanone); METHYL CEDRYL KETONE (1-((1S,8aS)-1,4,4,6-tetramethyl-2,3,3a,4,5,8-hexahydro-1H-5,8a-methanoazulen-7-yl)ethanone); METHYL NONYL KETONE EXTRA (undecan-2-one); METHYL OCTYNE CARBONATE (methyl non-2-ynoate); METHYL PAMPLEMOUSSE (6,6-dimethoxy-2,5,5-trim ethylhex-2-ene); MYRALDENE (4-(4-methylpent-3-en-1-yl)cyclohex-3-enecarbaldehyde); NECTARYL (2-(2-(4-methylcyclohex-3-en-1-yl)propyl)cyclopentanone); NEOBERGAMATE^{™} FORTE (2-methyl-6-methyleneoct-7-en-2-yl acetate); NEOFOLIONE^{™} ((E)-methyl non-2-enoate); NEROLIDYLE^{™} ((Z)-3,7,11-trimethyldodeca-1,6,10-trien-3-yl acetate); NERYL ACETATE HC ((Z)-3,7-dimethylocta-2,6-dien-1-yl acetate); NONADYL (6,8-dimethylnonan-2-ol); NONENAL-6-CIS ((Z)-non-6-enal); NYMPHEAL^{™} (3-(4-isobutyl-2-methylphenyl)propanal); ORIVONE^{™} (4-(tert-pentyl)cyclohexanone); PARADISAMIDE^{™} (2-ethyl-N-methyl-N-(m-tolyl)butanamide); PELARGENE (2-methyl-4-methylene-6-phenyltetrahydro-2H-pyran); PEONILE^{™} (2-cyclohexylidene-2-phenylacetonitrile); PETALIA^{™} (2-cyclohexylidene-2-(o-tolyl) acetonit rile); PIVAROSE^{™} (2,2-dimethyl-2-pheylethyl propanoate); PRECYCLEMONE^{™} B (1-methyl-4-(4-methylpent-3-en-1-yl)cyclohex-3-enecarbaldehyde); PYRALONE^{™} (6-(sec-butyl)quinoline); RADJANOL^{™} SUPER ((E)-2-ethyl-4-(2,2,3-trimethylcyclopent-3-en-1-yl)but-2-en-1-ol); RASPBERRY KETONE (N112) (4-(4-hydroxyphenyl)butan-2-one); RHUBAFURANE^{™} (2,2,5-trim ethyl-5-pentylcyclopentanone); ROSACETOL (2,2,2-trichloro-1-phenylethyl acetate); ROSALVA (dec-9-en-1-ol); ROSYFOLIA ((1-methyl-2-(5-methylhex-4-en-2-yl)cyclopropyl)-methanol); ROSYRANE^{™} SUPER (4-methylene-2-phenyltetrahydro-2H-pyran); SERENOLIDE (2-(1-(3,3-dimethylcyclohexyl)ethoxy)-2-methylpropyl cyclopropanecarboxylate); SILVIAL^{™} (3-(4-isobutylphenyl)-2-methylpropanal); SPIROGALBANONE^{™} (1-(spiro[4.5]dec-6-en-7-yl)pent-4-en-1-one); STEMONE^{™} ((E)-5-methylheptan-3-one oxime); SUPER MUGUET^{™} ((E)-6-ethyl-3-methyloct-6-en-1-ol); SYLKOLIDE^{™} ((E)-2-((3,5-dimethylhex-3-en-2-yl)oxy)-2-methylpropyl cyclopropanecarboxylate); TERPINENE GAMMA (1-methyl-4-propan-2-ylcyclohexa-1,4-diene); TERPINOLENE (1-methyl-4-(propan-2-ylidene)cyclohex-1-ene); TERPINYL ACETATE (2-(4-methylcyclohex-3-en-1-yl)propan-2-yl acetate); TETRAHYDRO LINALOOL (3,7-dimethyloctan-3-ol); TETRAHYDRO MYRCENOL (2,6-dimethyloctan-2-ol); THIBETOLIDE (oxacyclohexadecan-2-one); TRIDECENE-2-NITRILE ((E)-tridec-2-enenitrile); UNDECAVERTOL ((E)-4-methyldec-3-en-5-ol); VELOUTONE^{™} (2,2,5-trimethyl-5-pentylcyclopentanone); VIRIDINE^{™} ((2,2-dimethoxyethyl)benzene); ZINARINE^{™} (2-(2,4-dimethylcyclohexyl)pyridine); and mixtures thereof.

A comprehensive list of perfume ingredients that may be encapsulated in accordance with the present invention can be found in the perfumery literature, for example *"*Perfume & Flavor Chemicals", S. Arctander, Allured Publishing, 2000.

The at least one benefit agent can also be a cosmetic ingredient. Preferably, the cosmetic ingredients have a calculated octanol/water partition coefficient (ClogP) of 1.5 or more, more preferably 3 or more. Alternatively preferred, the ClogP of the cosmetic ingredient is from 2 to 7.

Particularly useful cosmetic ingredients may be selected from the group consisting of emollients, smoothening actives, hydrating actives, soothing and relaxing actives, decorative actives, anti-aging actives, draining actives, remodeling actives, skin levelling actives, preservatives, anti-oxidant actives, antibacterial or bacteriostatic actives, cleansing actives, lubricating actives, structuring actives, hair conditioning actives, whitening actives, texturing actives, softening actives, anti-dandruff actives and exfoliating actives.

Particularly useful cosmetic ingredients include, but are not limited to, hydrophobic polymers, such as alkyldimethylsiloxanes, polymethyl silsesquioxanes, polyethylene, polyisobutylene, styrene-ethylene-styrene and styrene-butylene-styrene block copolymers, mineral oils, such as hydrogenated isoparaffins, silicone oils, vegetable oils, such as argan oil, jojoba oil, aloe vera oil, fatty acids and fatty alcohols and their esters, glycolipides, phospholipides, sphingolipides, such as ceramides, sterols and steroids, terpenes, sesquiterpenes, triterpenes and their derivatives, essential oils, such as arnica oil, artemisia oil, bark tree oil, birch leaf oil, calendula oil, cinnamon oil, echinacea oil, eucalyptus oil, ginseng oil, jujube oil, helianthus oil, jasmine oil, lavender oil, lotus seed oil, perilla oil, rosmary oil, sandal wood oil, tea tree oil, thyme oil, valerian oil, wormwood oil, ylang ylang oil, yucca oil.

Core-shell microcapsules according to the present invention generally have a volume average size (d50) of 1 to 100 µm, preferably 5 to 50 µm, even more preferably 10 to 20 µm. The volume-average size of dispersed particles, such as microcapsules, may be obtained by light scattering measurements according to methods known to the skilled person.

A further aspect of the present invention relates to a method for preparing an encapsulated composition, in particular an encapsulated composition as described herein above. The method comprises the steps of:
a) Providing a core composition comprising at least one trifunctional isocyanate, optionally a water dispersible polyisocyanate, and at least one benefit agent;
b) Mixing the core composition with water and optionally a cationic copolymer;
c) Emulsifying the mixture obtained in b) in order to obtain core composition droplets having a volume average size of 1 to 100 µm, preferably 5 to 50 µm, even more preferably 10 to 20 µm ;
d) Adding at least one polyfunctional amine not being chitosan, preferably while keeping the mixture obtained in c) under stirring;
e) Heating progressively the mixture obtained in d) to a temperature of from 60 to 95 °C, preferably from 80 to 90 °C, preferably over a period of time from 2 hours to 4 hours, for example 3 ± 0.5 hours;
f) Adding chitosan to the mixture obtained in step e), and preferably maintaining the mixture at the temperature applied in step e), in particular over a period of time from 1 to 5 hours, for example 2 ± 0.5 hours or 4.5 ± 0.5 hours;
g) Letting the mixture obtained in f) cool down to room temperature, in order to obtain a slurry of microcapsules.

The weight of the trifunctional isocyanate used in step a) in relation to the weight of the core composition provided in step a) is between 8 and 20 wt.-%, preferably between 9 and 16 wt.-%, more preferably between 10 and 12 wt.-%.

Oil-in-water emulsions have the advantage of providing a plurality of droplets that may be used as a template for shell formation, wherein the shell is built around each of these droplets. Additionally, the droplet size distribution may be controlled in emulsions, by controlling the conditions of emulsifications, such as stirring speed and stirrer geometry. As a result, a plurality of microcapsules is obtained with controlled average size and size distribution, wherein the oil phase is encapsulated and forms thereby the core of the microcapsules. In a particular embodiment of the present invention, the mixture formed in step b) is allowed to stand without stirring until the core composition and the water phase separate.

The weight of the water dispersible polyisocyanate optionally used in step a) in relation to the weight of the core composition provided in step a) can be between 0.1 and 20 wt.-%, preferably between 0.5 and 10 wt.-%, more preferably between 1 and 5 wt.-%.The weight of the at least one polyfunctional amine not being chitosan used in step d) in relation to the weight of the mixture obtained in step f) can be between 0.1 and 2 wt.-%, preferably between 0.25 and 1 wt.-%, more preferably between 0.4 and 0.8 wt.-%.

The weight of the cationic copolymer optionally used in step b) in relation to the weight of the mixture obtained in step f) can be between 2 and 10 wt.-%, preferably between 2.5 and 7.5 wt.-%, more preferably between 3 and 5 wt.-%.

The weight of the chitosan used in step f) in relation to the weight of the mixture obtained in step f) can be between 0.06 and 0.3 wt.-%, preferably between 0.12 and 0.24 wt.-%, more preferably between 0.18 and 0.21 wt.-%.

The appropriate stirring speed and geometry of the mixer can be selected in order to obtain the desired average droplet size and droplet size distribution. In a method according to the present invention, a one-liter vessel equipped with a turbine, or a cross-beam stirrer with pitched beam, such as a Mig stirrer, and having a stirrer diameter to reactor diameter of 0.6 to 0.8 may be used. Microcapsules can be formed in such a reactor having a volume average size (d50) of 30 microns or less, more particularly 20 microns or less, at a stirring speed from about 100 to about 1200 rpm, more particularly from about 600 to 1000 rpm. Preferably, a Mig stirrer is used operating at a speed of 850 +/- 50 rpm. The person skilled in the art will however easily understand that such stirring conditions may change depending on the size of the reactor and of the batch size, on the exact geometry of the stirrer on the ratio of the diameter of the stirrer to the diameter of the reactor diameter ratios. For example, for a Mig stirrer with stirrer to reactor diameter ratio from 0.5 to 0.9 and slurry volumes ranging from 0.5 to 8 tons, the preferable agitation speed in the context of the present invention is from 150 rpm to 50 rpm.

After formation of the microcapsules, the encapsulated composition is usually allowed to cool down room temperature (cf. above step g)). Before, during or after cooling, the encapsulated composition may be further processed. Further processing may include treatment of the composition with anti-microbial preservatives, which preservatives are well known in the art. Further processing may also include the addition of a suspending aid, such as a hydrocolloid suspending aid to assist in the stable physical dispersion of the microcapsules and prevent any creaming or coalescence. A preferred suspending aid is hydroxyethyl cellulose, for instance Natrosol 250HX (ex Ashland). Any additional adjuvants conventional in the art may also be added during further-processing.

A further aspect of the present invention relates to an encapsulated composition obtainable by the method described herein above.

Yet another aspect of the present invention relates to a use of an encapsulated composition as described herein above to enhance the performance of a benefit agent in a consumer product.

The present invention also relates to a consumer product comprising an encapsulated composition as described herein above. The consumer product is preferably a hair care product, in particular selected from the group consisting of a shampoo and a hair conditioner. But, on the other hand, the consumer product can also be other kind of personal care product, such as a soap, a body wash, a shower gel or a skin care product.

As used herein, a *"consumer product"* means an article intended to be used or consumed in the form in which it is sold, and not intended for subsequent commercial manufacture or modification.

Encapsulated compositions according to the present invention are particularly useful when employed as perfume delivery vehicles in consumer products that require, for delivering optimal perfumery benefits, that the microcapsules adhere well to a substrate on which they are applied. Such consumer products include in particular hair shampoos and conditioners, but also textile-treatment products, such as laundry detergents and conditioners.

A consumer product according to the present invention can comprise from 0.01 to 5 wt.-%, preferably from 0.05 to 2.5 wt.-%, more preferably from 0.1 to 1 wt.-% microcapsules as described herein above, referred to the total weight of the consumer product.

A consumer product according to the present invention can comprise from 0.00001 to 0.005 wt.-%, more particularly from 0.00005 to 0.001 wt.-%, still more particularly from 0.00008 to 0.0005 wt.-% free chitosan, referred to the total weight of the consumer product.

The present disclosure also relates to a method for enhancing the performance of a benefit agent in a consumer product by adding an encapsulated composition according to the present invention.

Particular features and further advantages of the present invention become apparent from the following examples.

### Example 1 - Preparation of polyurea microcapsules according to the present invention

In Example 1.1, the microcapsules have been obtained by performing the steps of:
- Preparing a core composition by admixing 1 g of Bayhydur XP 2547 (ex Covestro), 4 g of Takenate D110N (ex Mitsui) and 30 g of perfume composition (ex Givaudan) in a reactor equipped with a stirrer;
- Stirring the core composition for 10 minutes at 600 rpm;
- Adding 40 g of water to the core composition and then adding 3 g of poly(methacrylamidopropyltrimethylammonium chloride-co-acrylic acid) copolymer having 71 mol-% methacrylamidopropyltrimethylammonium chloride and 29 mol-% acrylic acid, prepared according to WO 2016/207187 A1 (Example 1);
- Stirring the mixture obtained at 1100 rpm for 30 minutes in order to obtain an emulsion with a volume-average core composition droplet size (d50) of 10 ± 2 µm;
- Adding 0.6 g of Lupasol G100, (ex BASF);
- Heating the mixture at a rate of 0.3 °C/min;
- After 120 minutes, adding a mixture of 0.2 g chitosan with an average molecular weight of 200'000 g/mol (from shrimps, ex Acros), 0.4 g of 30 vol.-% acetic acid in water, and 10 g of water;
- Once the temperature of 85 °C has been reached, keeping the mixture for an additional 120 minutes at this temperature;
- Adding 4 g of a 20 % ammonia solution in water and keeping the mixture for an additional 35 minutes;
- Letting the resulting mixture cool to 25 °C within 60 minutes, in order to obtain a slurry of microcapsules.

The solid content of the slurry was measured by using a thermo-balance operating at 120 °C. The solid content, expressed as weight percentage of the initial slurry deposited on the balance, was taken at the point where the drying-induced rate of weight change had dropped below 0.1 %/min. The ratio of the measured solid content to the theoretical solid content calculated based on the weight of perfume and encapsulating materials involved is taken as a measurement of encapsulation yield, expressed in wt.-%.

The solid content of the slurry obtained was 37 wt.-%, the volume average size (d50) of the capsules was 10 ± 1 µm and the encapsulation efficiency was 100 %.

In Example 1.1, the weight of the trifunctional isocyanate used in relation to the weight of the core composition provided was 11 wt.-% (Trifunctional isocyanate to perfume weight ratio: 0.13).

In Example 1.2, the microcapsules have been obtained by using the same process as in Example 1.1, but the weight of the trifunctional isocyanate used in relation to the weight of the core composition provided was 14 wt.-% (Trifunctional isocyanate to perfume weight ratio: 0.17).

In Example 1.3, the microcapsules have been obtained by using the same process as in Example 1.1, but the weight of the trifunctional isocyanate used in relation to the weight of the core composition provided was 19 wt.-% (Trifunctional isocyanate to perfume weight ratio: 0.25).

In Example 1.4, the microcapsules have been obtained by using the same process as in Example 1.1, but with mushroom chitosan having a molecular weight of 85'000 g/mol (ex Kionutrime).

In Example 1.5, the microcapsules have been obtained by using the same process as in Example 1.1, but with mushroom chitosan having a molecular weight of 15'000 g/mol (ex Kionutrime).

**Table 1: Variations of polyisocyanate and chitosan types**

| | Trifunctional isocyanate (Takenate D110N) to perfume weight ratio | Type of chitosan |
|---|---|---|
| EXAMPLE 1.1 | 0.13 | Shrimp (200'000 g/mol) |
| EXAMPLE 1.2 | 0.17 | Shrimp (200'000 g/mol) |
| EXAMPLE 1.3 | 0.25 | Shrimp (200'000 g/mol) |
| EXAMPLE 1.4 | 0.13 | Mushroom (85'000 g/mol) |
| EXAMPLE 1.5 | 0.13 | Mushroom (15'000 g/mol) |

### Example 2 - Comparative examples

A series of microcapsules have been obtained by performing the process described in Example 1.1, wherein the Takenate D110N to perfume oil weight ratio and chitosan to perfume oil weight ratio have been varied according to Table 2.

In example 2.1 and 2.2, Takenate D110N was replaced by Desmodur W (methylenebis(4-cyclohexylisocyanate)).

**Table 2: Variations of polyisocyanate and chitosan amounts**

| | Trifunctional isocyanate (Takenate D11 0N) to perfume weight ratio | Trifunctional isocyanate (Desmodur W) to perfume weight ratio | Chitosan to perfume ratio |
|---|---|---|---|
| EXAMPLE 2.1 | 0 | 0.13 | 0 |
| EXAMPLE 2.2 | 0 | 0.13 | 0.0067 |
| EXAMPLE 2.3 | 0.08 | 0 | 0 |
| EXAMPLE 2.4 | 0.08 | 0 | 0.0067 |
| EXAMPLE 2.5 | 0.13 | 0 | 0 |

The reference samples 2.1 and 2.2 were not stable over storage, with 100% perfume leakage in shampoo base.

### Example 3 - Comparison of olfactive performance

The olfactive performance of the microcapsules was assessed by a panel of four experts who rated the odor intensity on a scale of 1-5 (1 = barely noticeable, 2 = weak, 3 = medium, 4 = strong and 5 = very strong).

The samples were evaluated in unperfumed shampoo and in unperfumed hair care conditioner. The aforementioned slurries were added to these bases under gentle stirring with a paddle mixer, so that the level of slurry was 0.6 wt.-%, referred to the total weight of each base.

In the case of shampoos, 20 wt.-% of shampoo, based on the total weight of hair swatches, was applied on the swatches that were previously humidified with the same weight of water. The dry weight of each swatch was about 12 g.

In the case of hair conditioners, the swatches were previously washed with an unperfumed shampoo and rinsed with water. Then, 10 wt.-% of conditioner, based on the total weight of the swatches, was applied just after washing with the shampoo.

For both shampoos and hair conditioners, the swatches were submitted to a massage 20 sec and then rinsed 30 seconds under running tap water at 37 °C at a flow rate of 3.2 l/min, without touching the swatch by hand. For this evaluation, the swatches were handled carefully in order to minimize the risk of breaking the microcapsules mechanically. The pre-rub and post-rub olfactive evaluation was performed after drying the swatches for 24 h at room temperature (cold air dying). This evaluation was performed by gently combing one part of each swatch 5 times.

For evaluation involving hair dryer (hot hair drying), the hair swatches were let in air for 15 minutes. Then a standard hair dryer operating at an inner temperature of 180 °C and providing a volumetric air flow rate of 0.016 m³/s was placed at 5 cm apart from the hair swatch and the air flow was applied for 5 minutes while moving the air flow up and down the swatch during these 5 minutes. The temperature on the hair was about 40-45 °C.

The results are shown in Table 3 and Table 4.

**Table 3: Olfactive performance on hair swatch - shampoo case**

| | Perfume impact on dry brushed hair, after hot drying with hair dryer | |
|---|---|---|
| | Fresh sample | Sample aged for 2 weeks at 37 °C |
| Reference microcapsules with Takenate D110N to perfume ratio of 0.08; Without chitosan (EXAMPLE 2.3) | 1.5 | n.d. |
| Reference microcapsules with Takenate D110N to perfume ratio of 0.13; Without chitosan (EXAMPLE 2.5) | 1.3 | n.d. |
| Microcapsules with Takenate D110N to perfume ratio of 0.13; Shrimp chitosan 200'000 g/mol (EXAMPLE 1.1) | 2.5 | n.d. |
| Microcapsules with Takenate D110N to perfume ratio of 0.13; Mushroom chitosan 85'000 g/mol (EXAMPLE 1.4) | 2.5 | 2.2 |
| Microcapsules with Takenate D110N to perfume ratio of 0.13; Mushroom chitosan 15'000 g/mol (EXAMPLE 1.5) | 2.5 | 2.3 |

**Table 4: Olfactive performance on hair swatch of freshly prepared and aged microcapsules - hair conditioner case**

| | Perfume impact on dry brushed hair, after drying with hair dryer | |
|---|---|---|
| | Fresh sample | Sample aged for 2 weeks at 37 °C |
| Reference microcapsules with Takenate D110N to perfume ratio of 0.08; Without chitosan (EXAMPLE 2.3) | 1.7 | n.d. |
| Reference microcapsules with Takenate D110N to perfume ratio of 0.13; Without chitosan (EXAMPLE 2.5) | 1.3 | n.d. |
| Microcapsules with Takenate D110N to perfume ratio of 0.08; Shrimp chitosan 200'000 g/mol (EXAMPLE 2.4) | 1.3 | n.d. |
| Microcapsules with Takenate D110N to perfume ratio of 0.13; Shrimp chitosan 200'000 g/mol (EXAMPLE 1.1) | 2.9 | n.d. |
| Microcapsules with Takenate D110N to perfume ratio of 0.17; chitosan (EXAMPLE 1.2) | 2.5 | 2.0 |
| Microcapsules with Takenate D110N to perfume ratio of 0.13; Mushroom chitosan 85'000 g/mol (EXAMPLE 1.4) | 2.0 | 1.9 |

These results demonstrate the positive impact of combining Takenate D110N with chitosan on retaining the performance of microcapsules after hair drying with a hair drier, even after storage in shampoo and conditioner bases.

## Claims

1. An encapsulated composition comprising at least one core-shell microcapsule, wherein the at least one core-shell microcapsule comprises a core comprising at least one benefit agent and a shell surrounding the core, wherein the shell comprises a polyurea resin formed by reaction of at least one trifunctional isocyanate with at least one polyfunctional amine not being chitosan, wherein the at least one trifunctional isocyanate is an adduct of an aliphatic triol with at least one araliphatic diisocyanate, wherein the weight ratio between moieties of the polyurea resin, which are derived from the trifunctional isocyanate, and the core is between 0.09 and 0.30, preferably between 0.10 and 0.20, more preferably between 0.11 and 0.18, and wherein the shell additionally comprises chitosan.

2. The composition according to claim 1, wherein
a) the aliphatic triol is selected from the group consisting of 2-ethylpropane-1,2,3-triol and 2-ethyl-2-(hydroxymethyl)propane-1,3-diol, preferably 2-ethylpropane-1,2,3-triol; and/or
b) the at least one araliphatic diisocyanate is selected from the group consisting of 1,2-bis(isocyanatomethyl)benzene, 1,3-bis(isocyanatomethyl)benzene, 1,4-bis(isocyanatomethyl)benzene, 1-isocyanato-2-(isocyanatomethyl)benzene, 1-isocyanato-3-(isocyanatomethyl) benzene and 1-isocyanato-4-(isocyanatom ethyl) benzene.

3. The composition according to claim 1, wherein the at least one trifunctional isocyanate is an adduct of 2-ethylpropane-1,2,3-triol and 1,3-bis(isocyanatomethyl)benzene.

4. The composition according to one of claims 1 to 3, wherein the chitosan is deposited on an outer surface of the shell surrounding the core and/or the chitosan is entrapped in the shell surrounding the core, optionally wherein the composition additionally comprises free chitosan.

5. The composition according to one of claims 1 to 3, wherein the polyurea resin is formed by reaction of the at least one trifunctional isocyanate with the at least one polyfunctional amine not being chitosan and with the chitosan.

6. The composition according to one of claims 1 to 5, wherein the polyurea resin is formed by reaction of the at least one trifunctional isocyanate and a water-dispersible polyisocyanate with the at least one polyfunctional amine not being chitosan, and optionally with the chitosan.

7. The composition of according to one of claims 1 to 6, wherein
a) the water-dispersible polyisocyanate is a water-dispersible polyisocyanate based on hexamethylene diisocyanate; and/or
b) the polyfunctional amine not being chitosan is a poly(ethyleneimine).

8. The composition according to one of claims 1 to 7, wherein the shell additionally comprises a cationic polymer.

9. The composition according to one of claims 1 to 8, wherein the weight ratio between the chitosan and/or moieties of the polyurea resin, which are derived from the chitosan, and the core is between 0.002 and 0.01, preferably between 0.004 and 0.008, more preferably between 0.006 and 0.007.

10. The composition according to one of claims 1 to 9, wherein the chitosan has
a) an average molecular weight of from 5'000 to 1'000'000 g/mol, preferably from 7'500 to 500'000 g/mol, still more preferably from 10'000 to 250'000 g/mol; and/or
b) an average deacetylation grade of higher than 60 %, more particularly higher than 70 %, still more particularly higher than 80 %.

11. The composition according to one of claims 1 to 10, wherein the benefit agent is selected from the group consisting of at least one perfume ingredient and at least one cosmetic ingredient.

12. A method for preparing an encapsulated composition according to one of claims 1 to 11, the method comprising the steps of:
a) Providing a core composition comprising at least one trifunctional isocyanate, optionally a water dispersible polyisocyanate, and at least one benefit agent;
b) Mixing the core composition with water and optionally a cationic copolymer;
c) Emulsifying the mixture obtained in b) in order to obtain core composition droplets having a volume average size of 1 to 100 µm, preferably 5 to 50 µm, even more preferably 10 to 20 µm, as measured by light scattering ;
d) Adding at least one polyfunctional amine not being chitosan, preferably while keeping the mixture obtained in c) under stirring;
e) Heating progressively the mixture obtained in d) to a temperature of from 60 to 95 °C, preferably from 80 to 90 °C, preferably over a period of time from 2 hours to 4 hours, for example 3 ± 0.5 hours;
f) Adding chitosan to the mixture obtained in step e), and preferably maintaining the mixture at the temperature applied in step e), in particular over a period of time from 1 to 5 hours, for example 2 ± 0.5 hours or 4.5 ± 0.5 hours;
g) Letting the mixture obtained in f) cool down to room temperature, in order to obtain a slurry of microcapsules.

13. The method according to claim 12, wherein the mixture formed in step b) is allowed to stand without stirring until the core composition and the water phase separate.

14. Use of an encapsulated composition according to one of claims 1 to 11 to enhance the performance of a benefit agent in a consumer product.

15. A consumer product comprising an encapsulated composition according to one of claims 1 to 11, wherein the consumer product is preferably a hair care product, in particular selected from the group consisting of a shampoo and a hair conditioner.

## Patentansprüche

1. Verkapselte Zusammensetzung umfassend wenigstens eine Kern-Schale-Mikrokapsel, wobei die wenigstens eine Kern-Schale-Mikrokapsel einen Kern, der wenigstens einen Wirkstoff umfasst, und eine Schale, die den Kern umgibt, umfasst, wobei die Schale ein Polyharnstoffharz umfasst, das durch Umsetzung wenigstens eines trifunktionellen Isocyanats mit wenigstens einem polyfunktionellen Amin, das nicht Chitosan ist, gebildet ist, wobei das wenigstens eine trifunktionelle Isocyanat ein Addukt eines aliphatischen Triols mit wenigstens einem araliphatischen Diisocyanat ist, wobei das Gewichtsverhältnis zwischen Einheiten des Polyharnstoffharzes, die von dem trifunktionellen Isocyanat abgeleitet sind, und dem Kern zwischen 0,09 und 0,30 beträgt, vorzugsweise zwischen 0,10 und 0,20, bevorzugter zwischen 0,11 und 0,18, und wobei die Schale zusätzlich Chitosan umfasst.

2. Zusammensetzung gemäß Anspruch 1, wobei
a) das aliphatische Triol ausgewählt ist aus der Gruppe bestehend aus 2-Ethylpropan-1,2,3-triol und 2-Ethyl-2-(hydroxymethyl)propan-1,3-diol, vorzugsweise 2-Ethylpropan-1,2,3-triol; und/oder
b) das wenigstens eine araliphatische Diisocyanat ausgewählt ist aus der Gruppe bestehend aus 1,2-Bis(isocyanatomethyl)benzol, 1,3-Bis(isocyanatomethyl)benzol, 1,4-Bis(isocyanatomethyl)benzol, 1-Isocyanato-2-(isocyanatomethyl)benzol, 1-Isocyanato-3-(isocyanatomethyl)benzol und 1-Isocyanato-4-(isocyanatomethyl)benzol.

3. Zusammensetzung gemäß Anspruch 1, wobei das wenigstens eine trifunktionelle Isocyanat ein Addukt von 2-Ethylpropan-1,2,3-triol und 1,3-Bis(isocyanatomethyl)benzol ist.

4. Zusammensetzung gemäß einem der Ansprüche 1 bis 3, wobei das Chitosan auf einer Außenoberfläche der Schale, die den Kern umgibt, abgeschieden ist und/oder das Chitosan in der Schale, die den Kern umgibt, eingeschlossen ist, gegebenenfalls wobei die Zusammensetzung zusätzlich freies Chitosan umfasst.

5. Zusammensetzung gemäß einem der Ansprüche 1 bis 3, wobei das Polyharnstoffharz durch Umsetzung des wenigstens einen trifunktionellen Isocyanats mit dem wenigstens einem polyfunktionellen Amin, das nicht Chitosan ist, und mit dem Chitosan gebildet ist.

6. Zusammensetzung gemäß einem der Ansprüche 1 bis 5, wobei das Polyharnstoffharz durch Umsetzung des wenigstens einen trifunktionellen Isocyanats und eines wasserdispergierbaren Polyisocyanats mit dem wenigstens einem polyfunktionellen Amin, das nicht Chitosan ist, und gegebenenfalls mit dem Chitosan gebildet ist.

7. Zusammensetzung gemäß einem der Ansprüche 1 bis 6, wobei
a) das wasserdispergierbare Polyisocyanat ein wasserdispergierbares Polyisocyanat auf der Basis von Hexamethylendiisocyanat ist; und/oder
b) das polyfunktionelle Amin, das nicht Chitosan ist, ein Poly(ethylenimin) ist.

8. Zusammensetzung gemäß einem der Ansprüche 1 bis 7, wobei die Schale zusätzlich ein kationisches Polymer umfasst.

9. Zusammensetzung gemäß einem der Ansprüche 1 bis 8, wobei das Gewichtsverhältnis zwischen dem Chitosan und/oder Einheiten des Polyharnstoffharzes, die von dem Chitosan abgeleitet sind, und dem Kern zwischen 0,002 und 0,01 beträgt, vorzugsweise zwischen 0,004 und 0,008, bevorzugter zwischen 0,006 und 0,007.

10. Zusammensetzung gemäß einem der Ansprüche 1 bis 9, wobei das Chitosan aufweist:
a) ein mittleres Molekulargewicht von 5.000 bis 1.000.000 g/mol, vorzugsweise von 7.500 bis 500.000 g/mol, noch bevorzugter von 10.000 bis 250.000 g/mol; und/oder
b) einen mittleren Deacetylierungsgrad von höher als 60 %, insbesondere höher als 70 %, besonders höher als 80 %.

11. Zusammensetzung gemäß einem der Ansprüche 1 bis 10, wobei der Wirkstoff ausgewählt ist aus der Gruppe bestehend aus wenigstens einem Parfüminhaltsstoff und wenigstens einem kosmetischen Inhaltsstoff.

12. Verfahren zur Herstellung einer verkapselten Zusammensetzung gemäß einem der Ansprüche 1 bis 11, wobei das Verfahren die Schritte umfasst:
a) Bereitstellen einer Kernzusammensetzung, die wenigstens ein trifunktionelles Isocyanat, gegebenenfalls ein wasserdispergierbares Polyisocyanat und wenigstens einen Wirkstoff umfasst;
b) Mischen der Kernzusammensetzung mit Wasser und gegebenenfalls einem kationischen Copolymer;
c) Emulgieren des bei b) erhaltenen Gemischs, um Kernzusammensetzungströpfchen mit einer volumengemittelten Größe von 1 bis 100 µm, vorzugsweise 5 bis 50 µm, bevorzugter 10 bis 20 µm, wie gemessen durch Lichtstreuung, zu erhalten;
d) Zugeben wenigstens eines polyfunktionellen Amins, das nicht Chitosan ist, vorzugsweise unter Halten des bei c) erhaltenen Gemischs unter Rühren;
e) fortschreitendes Erhitzen des bei d) erhaltenen Gemischs auf eine Temperatur von 60 bis 95 °C, vorzugsweise von 80 bis 90 °C, vorzugsweise über einen Zeitraum von 2 Stunden bis 4 Stunden, beispielsweise 3 ± 0,5 Stunden;
f) Zugeben von Chitosan zu dem bei Schritt e) erhaltenen Gemisch und vorzugsweise Halten des Gemischs bei der bei Schritt e) angewendeten Temperatur, insbesondere über einen Zeitraum von 1 bis 5 Stunden, beispielsweise 2 ± 0,5 Stunden oder 4,5 ± 0,5 Stunden;
g) Abkühlenlassen des bei f) erhaltenen Gemischs auf Raumtemperatur, um eine Aufschlämmung von Mikrokapseln zu erhalten.

13. Verfahren gemäß Anspruch 12, wobei das bei Schritt b) gebildete Gemisch ohne Rühren stehen gelassen wird, bis sich die Kernzusammensetzung und die Wasserphase trennen.

14. Verwendung einer verkapselten Zusammensetzung gemäß einem der Ansprüche 1 bis 11 zum Erhöhen der Leistungsfähigkeit eines Wirkstoffs in einem Verbraucherprodukt.

15. Verbraucherprodukt umfassend eine verkapselte Zusammensetzung gemäß einem der Ansprüche 1 bis 11, wobei das Verbraucherprodukt vorzugsweise ein Haarpflegeprodukt ist, insbesondere ausgewählt aus der Gruppe bestehend aus einem Shampoo und einer Haarspülung.

## Revendications

1. Composition encapsulée comprenant au moins une microcapsule de type coeur-écorce, dans laquelle l'au moins une microcapsule de type cœur-écorce comprend un coeur comprenant au moins un agent bénéfique et une écorce entourant le coeur, dans laquelle l'écorce comprend une résine de polyurée formée par réaction d'au moins un isocyanate trifonctionnel avec au moins une amine polyfonctionnelle qui n'est pas le chitosane, dans laquelle l'au moins un isocyanate trifonctionnel est un adduit d'un triol aliphatique avec au moins un diisocyanate arylaliphatique, dans laquelle le rapport pondéral entre les fractions de la résine de polyurée qui sont dérivées de l'isocyanate trifonctionnel et le coeur est compris entre 0,09 et 0,30, de préférence entre 0,10 et 0,20, plus préférablement entre 0,11 et 0,18, et dans laquelle l'écorce comprend de plus du chitosane.

2. Composition selon la revendication 1, dans laquelle
a) le triol aliphatique est choisi dans le groupe constitué par le 2-éthylpropane-1,2,3-triol et le 2-éthyl-2-(hydroxyméthyl)propane-1,3-diol, de préférence il est le 2-éthylpropane-1,2,3-triol ; et/ou
b) l'au moins un diisocyanate arylaliphatique est choisi dans le groupe constitué par le 1,2-bis(isocyanatométhyl)benzène, le 1,3-bis(isocyanatométhyl)benzène, le 1,4-bis(isocyanatométhyl)benzène, le 1-isocyanato-2-(isocyanatométhyl)benzène, le 1-isocyanato-3-(isocyanatométhyl)benzène et le 1-isocyanato-4-(isocyanatométhyl)benzène.

3. Composition selon la revendication 1, dans laquelle l'au moins un isocyanate trifonctionnel est un adduit de 2-éthylpropane-1,2,3-triol et de 1,3-bis(isocyanatométhyl)benzène.

4. Composition selon l'une des revendications 1 à 3, dans laquelle le chitosane est déposé sur une surface externe de l'écorce entourant le coeur et/ou le chitosane est piégé dans l'écorce entourant le coeur, éventuellement la composition comprenant de plus du chitosane libre.

5. Composition selon l'une des revendications 1 à 3, dans laquelle la résine de polyurée est formée par réaction de l'au moins un isocyanate trifonctionnel avec l'au moins une amine polyfonctionnelle qui n'est pas du chitosane et avec le chitosane.

6. Composition selon l'une des revendications 1 à 5, dans laquelle la résine de polyurée est formée par réaction de l'au moins un isocyanate trifonctionnel et d'un polyisocyanate dispersible dans l'eau avec l'au moins une amine polyfonctionnelle qui n'est pas le chitosane et éventuellement avec le chitosane.

7. Composition selon l'une des revendications 1 à 6, dans laquelle
a) le polyisocyanate dispersible dans l'eau est un polyisocyanate dispersible dans l'eau à base de diisocyanate d'hexaméthylène ; et/ou
b) l'amine polyfonctionnelle qui n'est pas le chitosane est une poly(éthylèneimine).

8. Composition selon l'une des revendications 1 à 7, dans laquelle l'écorce comprend de plus un polymère cationique.

9. Composition selon l'une des revendications 1 à 8, dans laquelle le rapport pondéral entre le chitosane et/ou les fractions de la résine de polyurée qui sont dérivées du chitosane et le coeur est compris entre 0,002 et 0,01, de préférence entre 0,004 et 0,008, plus préférablement entre 0,006 et 0,007.

10. Composition selon l'une des revendications 1 à 9, dans laquelle le chitosane a
a) une masse moléculaire moyenne allant de 5 000 à 1 000 000 g/mol, de préférence de 7 500 à 500 000 g/mol, encore plus préférablement de 10 000 à 250 000 g/mol ; et/ou
b) un taux moyen de désacétylation supérieur à 60 %, plus particulièrement supérieur à 70 %, encore plus particulièrement supérieur à 80 %.

11. Composition selon l'une des revendications 1 à 10, dans laquelle l'agent bénéfique est choisi dans le groupe constitué par au moins un ingrédient de parfum et au moins un ingrédient cosmétique.

12. Procédé pour la préparation d'une composition encapsulée selon l'une des revendications 1 à 11, le procédé comprenant les étapes consistant à :
a) fournir une composition de coeur comprenant au moins un isocyanate trifonctionnel, éventuellement un polyisocyanate dispersible dans l'eau, et au moins un agent bénéfique ;
b) mélanger la composition de coeur avec de l'eau et éventuellement un copolymère cationique ;
c) émulsifier le mélange obtenu en b) afin d'obtenir des gouttelettes de composition de coeur ayant une taille moyenne en volume, telle que mesurée par diffusion de la lumière, de 1 à 100 µm, de préférence de 5 à 50 µm, encore plus préférablement de 10 à 20 µm ;
d) ajouter au moins une amine polyfonctionnelle qui n'est pas le chitosane, de préférence tout en maintenant le mélange obtenu en c) sous agitation ;
e) chauffer progressivement le mélange obtenu en d) à une température allant de 60 à 95 °C, de préférence de 80 à 90 °C, de préférence sur une durée allant de 2 heures à 4 heures, par exemple de 3 ± 0,5 heures ;
f) ajouter du chitosane au mélange obtenu à l'étape e), et de préférence maintenir le mélange à la température appliquée à l'étape e), en particulier sur une durée allant de 1 à 5 heures, par exemple de 2 ± 0,5 heures ou de 4,5 ± 0,5 heures ;
g) laisser le mélange obtenu en f) refroidir à température ambiante, afin d'obtenir une suspension épaisse de microcapsules.

13. Procédé selon la revendication 12, dans lequel le mélange formé à l'étape b) est laissé au repos sans agitation jusqu'à ce que la composition de coeur et la phase aqueuse se séparent.

14. Utilisation d'une composition encapsulée selon l'une des revendications 1 à 11 pour améliorer les performances d'un agent bénéfique dans un produit de grande consommation.

15. Produit de grande consommation comprenant une composition encapsulée selon l'une des revendications 1 à 11, le produit de grande consommation étant de préférence un produit de soins des cheveux, en particulier choisi dans le groupe constitué par un shampooing et un après-shampooing.
